(19) Europäisches Patentamt
European Patent Office
Office européen des brevets

(11) Publication number: 0 009 404
B1

(12) **EUROPEAN PATENT SPECIFICATION**

(45) Date of publication of patent specification: 22.02.84

(51) Int. Cl.³: A 61 K 7/00, A 61 K 9/10, B 01 F 3/08

(21) Application number: 79301954.8

(22) Date of filing: 20.09.79

(54) **Water-in-oil emulsions and process for preparing them.**

(30) Priority: 22.09.78 GB 3783078

(43) Date of publication of application:
02.04.80 Bulletin 80/7

(45) Publication of the grant of the patent:
22.02.84 Bulletin 84/8

(84) Designated Contracting States:
AT BE CH DE FR GB IT NL SE

(56) References cited:
BE - A - 623 673
DE - A - 1 948 152
FR - A - 1 382 068
FR - A - 2 258 165
FR - A - 2 273 514
LU - A - 58 143
US - A - 4 035 513
US - A - 4 040 857

The file contains technical information submitted after the application was filed and not included in this specification

(73) Proprietor: UNILEVER PLC
Unilever House Blackfriars P O Box 68
London EC4P 4BQ (GB)
(84) GB
(73) Proprietor: UNILEVER NV
Burgemeester s'Jacobplein 1 P.O. Box 760
NL-3000 DK Rotterdam (NL)
(84) BE CH DE FR IT NL SE AT

(72) Inventor: Berthod, Daniel Pierre Marie
12 rue Hegesippe-Moreau
F-78018 Paris (FR)
Inventor: Benzoni, Andre Jean Edouard
11 Boulevard Marx-Dormoy
F-93190 Livry-Gargan (FR)
Inventor: Gauthier, Jacques Rene Antoine
3 rue Sain-James
F-92200 Neuilly sur Seine (FR)

(74) Representative: Tonge, Robert James et al,
UNILEVER PLC Patents Division P.O. Box 68
Unilever House
London EC4P 4BQ (GB)

EP 0 009 404 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Water-in-oil emulsions and process for preparing them

The invention relates to high internal phase water-in-oil emulsions suitable for the preparation of cosmetic, pharmaceutical and other products and to methods for the preparation of such emulsions.

By "high internal phase emulsion" is meant an emulsion in which the volume of the internal phase occupies at least 80% of the total volume of the emulsion.

Water-in-oil emulsions, other than high internal phase emulsions, have been employed in the formulation of cosmetic and pharmaceutical ointments, emollient creams, lotions and the like. However, while such emulsions can impart desirable characteristics of water-repellancy to the skin and provide a means whereby fats, oils and waxes can be absorbed onto human skin, the use of such emulsions in skin treatment products has sometimes been precluded because they have been implicated in toxicity, skin irritancy or excessive greasiness in use. The production of instability has also limited the use of high internal phase water-in-oil emulsions in cosmetics and pharmaceutical products where storage stability is a pre-requisite.

A study was accordingly undertaken to examine the instability of these emulsions and to establish means whereby stability could be improved. It was found, for example, that high internal phase water-in-oil emulsions containing as skin benefit agents a water soluble salt such as an organic lactate or a metallic sulphate dissolved in the aqueous phase, are stable for no more than a few days. Syneresis of the internal phase or even separation of the oily and aqueous phases can then generally be seen as an accumulation of oil at the surface of the emulsion.

This problem has led to the screening of many substances in a search for a stabilising agent whose incorporation in a product based on a high internal phase emulsion would effectively extend the shelf-life of that product at normal storage temperatures.

Applicants did note the proposal of Kumano in US—A—4 035 513 that water-in-oil emulsifier compositions suitable for providing stable cosmetics such as creams comprise a partial ester of a polyhydric alcohol having at least 3 hydroxyl groups and a fatty acid, and an amino acid or a salt thereof. Kumano, however, is concerned with emulsions in which by using his special emulsifier composition, the internal aqueous phase could be increased from a conventional value which does not exceed 25% to form from 30 to 70%, preferably 50 to 70% by volume of the emulsion.

It has now been discovered that the problem of instability of the high internal phase emulsion (internal phase at least 80% of the total volume) on storage can be overcome by incorporation into the aqueous phase of a water-soluble amino acid, a water-soluble amino acid salt or a mixture of amino acids and salts thereof. The high internal phase emulsions so obtained have good storage characteristics in that the water and oil phases do not separate when the emulsion is stored for at least three months, even at a "tropical" temperature of 50°C, or when subjected repeatedly to freezing at a temperature of −20°C and thawing at ambient temperature of +20°C.

These high internal phase emulsions are accordingly suitable for use in the preparation of cosmetic and pharmaceutical products and the like, particularly for topical application to human skin, which avoid the aforementioned disadvantages in that they are non-toxic, non-irritating to the skin, not excessively greasy in use and which do not suffer from instability on storage.

Accordingly, the invention provides a cosmetically and pharmaceutically acceptable water-in-oil emulsion comprising an amino acid or salt thereof or a mixture of amino acids and salts thereof in addition to water, an oil and an emulsifier, characterised in that:

(i) the amino acid component is water-soluble and forms from 0.5 to 25% by weight of the emulsion
(ii) the water forms from 60 to 95% by weight of the emulsion
(iii) the oil is a non-polar branched chain mineral oil and forms from 2 to 15% by weight of the emulsion and
(iv) the emulsifier is a liquid emulsifier having an HLB value of from 1 to 7 and forms from 0.5 to 10% by weight; of the emulsion

the emulsion having an aqueous phase comprising the amino acid component and water, the aqueous phase forming from 80 to 97% by volume of the emulsion, and an oily phase comprising the oil and emulsifier, the oily phase forming from 3 to 20% by volume of the emulsion.

The invention also provides a process for preparing a high internal phase water-in-oil emulsion which comprises the steps of mixing a water soluble amino acid or a salt thereof or a mixture of amino acids or salt thereof with water to provide an aqueous phase; mixing a liquid emulsifier having an HLB value of from 1 to 7 with a branched-chain non-polar mineral oil to provide an oily phase; and homogenising the aqueous phase with the oily phase to provide a high internal phase water-in-oil emulsion.

The emulsion according to the invention consists of an internal phase which is aqueous and an external phase which is oily. It should be explained that water-in-oil emulsions which are not high internal phase emulsions usually consist of from about 1 to 74% by volume of an aqueous phase

dispersed in about 99 to 26% by volume of an oily phase. A water-in-oil emulsion consisting of 74% by volume aqueous phase and 26% by volume of oily phase represents the theoretical maximum packing volume concentration of rigid monodisperse spheres of water as the internal phase in oil as the external phase. Hence, water-in-oil emulsions containing more than 74% aqueous phase are high internal phase emulsions.

The water-in-oil emulsion of the invention comprises from 80 to 97% by volume of an aqueous phase and from 3 to 20% by volume of an oily phase respectively.

In order to obtain stable high internal phase water-in-oil emulsions, it has been shown necessary to select carefully the oil ingredient, the emulsifier and to employ a water-soluble amino acid component as a special emulsion stabiliser to ensure that the emulsions once made generally remain stable over an extended period of time.

The oil

The oil should preferably be liquid at room temperature (20°C) and should be cosmetically and pharmaceutically acceptable.

The oil should also be non-polar and should contain branched chain alkyl groups. The preferred oils are highly branched-chain mineral oils.

Examples of preferred oils are (in decreasing order of preference; the capitalised names are trade marks):

$C_{10}$ to $C_{12}$ isoparaffins such as ISOPAR L (Esso)
Polyisobutene such as PARLEAM (Nichiyu)
Squalane such as COSBIOL (Laserson & Sabetay)
Branched chain light paraffin oil such as LYTOL (Witko) or WM1 (BP)
Mineral oil such as MARCHOL 82 (Esso) or CARNATION OIL (Witko)

The quantity of oil in the emulsion is from 2 to 15% by weight of the emulsion.

If the emulsion contains less than 1% oil, it is generally not possible to obtain a stable water-in-oil emulsion, whereas if the emulsion contains more than 24% oil, then the emulsion will cease to exhibit the special properties and characteristics attributable to a high internal phase emulsion.

The emulsifier

The emulsifier should be liquid at room temperature (20°C) and be cosmetically and pharmaceutically acceptable.

The emulsifier should have an HLB value of from 1 to 7, preferably from 2 to 6.

Examples of suitable emulsifiers are (in decreasing order of preference; the capitalised names are trade marks).

| | HLB value |
|---|---|
| HOSTAPHAT KO 300N (mono-, di-, and triphosphoric esters of oleic acid) by Hoechst | 2.3 |
| CREMOPHOR WO/A (ether of glycerol and fatty alcohols) by BASF | 7 |
| IMWITOR 780K (glycerol monoisostearate) by Witco | 3.7 |
| ARLACEL 987 (sorbitan isostearate) by Atlas | 7 |
| BRIJ 92 (polyoxyethylene(2)oleyl ether) by Atlas | 4.9 |
| Triglycerol monooleate by PVO International | 4.0 |
| ARLACEL 80 (sorbitan monooleate) by Atlas | 4.3 |
| ARLACEL 83 (sorbitan sesquioleate) by Atlas | 7 |
| ARLACEL 85 (sorbitan trioleate) by Atlas | 1.8 |
| Decaglycerol tetraoleate by PVO International | 6.0 |
| Decaglycerol octaoleate by PVO International | 4.0 |
| SIMULSOL 92 (polyethoxylated(2)oleyl alcohol) by Produits Chimiques de la Montagne Noire | 6.7 |

The quantity of emulsifier in the emulsion is from 0.5 to 10%, preferably 2 to 5% by weight of the emulsion.

If the emulsion contains less than 0.5% of emulsifier, it is unlikely that the emulsion, if obtained, will remain stable on storage, whereas if the emulsion contains more than 10% of emulsifer, the stability of the emulsion can be adversely affected.

Amino acid

An amino acid or its salt, preferably its alkali metal salt or hydrohalide, or a mixture thereof, are employed as a stabiliser for the emulsion.

The amino acid should be cosmetically and pharmaceutically acceptable and they, or their salts, should be soluble in water.

Examples of suitable water soluble amino acids or amino acid salts are:

glutamic acid

aspartic acid
glycine
$\alpha$-alanine
$\beta$-alanine
serine
arginine hydrochloride
histidine hydrochloride
lysine hydrochloride
sodium aspartate
potassium aspartate
sodium glutamate
potassium glutamate

It is also possible to employ a protein hydrolysate as a source of amino acid.

The quantity of amino acid in the emulsion is from 0.5 to 25%, preferably 1.5 to 10% by weight of the emulsion.

If the emulsion contains less than 0.5% of amino acid, it is unlikely that the emulsion will remain stable on storage, whereas if the emulsion contains more than 25% amino acid, the stability of the emulsion can be adversely affected.

Water

The emulsion also comprises water. The quantity of water in the emulsion is from 60 to 95% by weight of the emulsion.

If the emulsion contains more than 97% of water, the stability of the emulsion on storage is likely to be poor and syneresis can occur.

Cosmetically and pharmaceutically active ingredients

The emulsion according to the invention can be employed as a vehicle for a wide variety of cosmetically or pharmaceutically active ingredients, particularly ingredients which have some beneficial effect when applied to the skin.

The emulsion thus provides a means whereby such active ingredients can be diluted, preserved, conveyed to and distributed on the skin surface at an appropriate concentration.

Examples of active ingredients include moisturisers, such as:
sodium pyrrolidone carboxylate
sodium lactate
lactic acid
triethanolamine lactate
orotic acid
inositol
sodium chloride
$\alpha$-hydroxy $C_6$ to $C_{10}$ carboxylic acids

sunscreen agents, such as:
ethyl hexyl p-methoxy cinnamate
p-amino benzoic acid
propoxylated(2)ethyl p-amino benzoate
2-hydroxy-4-n-octoxybenzophenone
dipropylene glycol salicylate
2,2',4,4'-tetrahydroxybenzophenone
2-hydroxy-4-methoxy benzophenone-5-sulphonic acid
ethylexyl-2-cyano-3,3-diphenyl acrylate

germicides, such as:
2-bromo-2-nitro propan-1,3-diol
cetyl pyridinium chloride
3,4,4'-trichlorocarbanilide
2,4,4'-trichloro-2'-hydroxydiphenyl ether
benzalkonium chloride
para hydroxy benzoic acid
dehydroacetic acid

deodorants, such as:
2-ethyl-1,3-hexane diol
zinc oxide

antiperspirants, such as:
- aluminium chlorhydrate
- aluminium chloride
- sodium aluminium chlorhydroxy lactate complex
- zirconyl chlorhydrate

healing agents, such as:
- magnesium sulphate
- zinc sulphate

Functional adjuncts

The emulsion according to the invention can also contain functional adjuncts for further controlling the properties of a pharmaceutical or cosmetic composition containing the emulsion. Functional adjuncts include:

antioxidants, such as
- tocopherol
- ascorbyl palmitate
- propyl gallate
- butylated hydroxy toluene
- butylated hydroxyanisole

propellants, such as:
- trichlorofluoro methane
- dichlorodifluoro methane
- dichlorotetrafluoro ethane
- monochlorodifluoro methane
- trichlorotrifluoro ethane
- propane
- butane
- isobutane
- dimethyl ether
- carbon dioxide

solvents, such as:
- ethyl alcohol
- 2-ethylhexanol
- ethylene carbonate
- propylene carbonate
- methylene chloride
- iso-propyl alcohol
- castor oil
- linear polyethoxylated methanol
- ethylene glycol monoethyl ether
- diethylene glycol monobutyl ether
- diethylene glycol monoethyl ether
- propoxylated butanol
- propoxylated oleyl alcohol
- butyl stearate
- butyl myristate

humectants, such as:
- glycerin
- 1,3-butylene glycol
- sorbitol
- sodium 2-pyrrolidone-5-carboxylate
- soluble collagen
- dibutyl phthalate
- gelatin
- polyglycerogen
- ethoxylated (10—20 moles) glucose
- propoxylated (10—20 moles) glucose

thickeners for the oily phase, such as:
- tetra alkyl and/or trialkyl aryl ammonium smectites

chemically modified magnesium aluminium silicate
organically modified montmorillonite clay
fumed silica
hydroxyethyl stearate amide

emollients, such as:
stearyl alcohol
glyceryl monoricinoleate
glyceryl monostearate
sulphated tallow
propylene glycol
mink oil
cetyl alcohol
stearyl stearate
isopropyl isostearate
dimethyl brassylate
stearic acid
isobutyl palmitate
isocetyl stearate
oleyl alcohol
myristyl stearate
isopropyl lanolate
isopropyl laurate
hexyl laurate
decyl oleate
di-isopropyl adipate
2-octadecanol
iso-cetyl alcohol
myristyl ethoxymyristate
cetyl palmitate
dimethylpolysiloxane
di-n-butyl sebacate
di-isopropyl sebacate
di-2-ethyl hexyl sebacate
2-ethyl hexyl palmitate
isononyl isononanoate
isodecyl isononanoate
isotridecyl isononanoate
2-ethyl hexyl palmitate
2-ethyl hexyl stearate
di-(2-ethyl-hexyl)adipate
di-(2-ethyl-hexyl)succinate
isopropyl myristate
isopropyl palmitate
isopropyl stearate
butyl stearate
glyceryl monostearate
polyethylene glycols
propylene glycol
triethylene glycol
lanolin
castor oil
acetylated lanolin alcohols
acetylated lanolin
petrolatum
isopropyl ester of lanolin fatty acids
mineral oils
butyl myristate
isostearic acid
palmitic acid
isopropyl linoleate
cetyl lactate
lauryl lactate
myristyl lactate
quaternised hydroxy alkyl aminogluconate

decyl oleate
isodecyl oleate
di-isopropyl adipate
2-ethyl-hexyl palmitate
isostearyl neopentanoate
myristyl myristate
di-isopropyl adipate
oleyl ethoxy myristate
di-glycol stearate
ethylene glycol monostearate

Preparation of emulsion

The high internal phase water-in-oil emulsions of the invention can be prepared by mixing the amino acid, or its salt or a mixture of amino acids or salts thereof, with water to provide an aqueous phase, then mixing the liquid emulsifier having an HLB value of from 1 to 7 with the mineral oil to provide an oily phase and subsequently homogenising from 80 to 97 parts by volume of the aqueous phase with from 3 to 20 parts by volume of the oily phase to provide the emulsion.

The amino acid or salt will be dissolved in water.

The importance of including a water soluble amino acid component in the water-in-oil emulsions of the invention is illustrated by the following comparative experiments.

Product forms

The compositions of the invention can be formulated as liquid, for example products such as lotions for use in conjuncton with applicators such as a roll-ball applicator or a spray device such as an aerosol can containing propellant or a container fitted with a pump to dispense the product. Alternatively, the compositions of the invention can be solid or semi-solid, for example powders, moulded sticks, creams or gels, for use in conjunction with an applicator such as a powder sifter or a stick applicator, or simply a tube or lidded jar.

Experiment 1

In this comparative experiment, a series of soft creams of the type intended for topical application to the skin as moisturising products were prepared.

The selected moisturising agent was the organic lactate, triethanolamine lactate (as a 50% solution at pH 5.5).

The selected water soluble amino acid salt was sodium glutamate.

Stability of each product was assessed by visual inspection at regular intervals during storage at temperatures of 20°, 42° and 50°C. Appearance of oil at the surface of the products was indicative of breakdown of the emulsion and the period of storage up to this point was recorded as the period of stability.

The products tested in this comparative experiment had the following formulation as shown in Table 1.

TABLE 1

| | % by weight | | | |
|---|---|---|---|---|
| Emulsifier (liquid having HLB: 1 to 7) | 3 | 3 | 3 | 3 |
| Oil (non-polar with branched chain alkyl groups | 15 | 15 | 15 | 15 |
| Sodium glutamate | — | — | 2.5 | 2.5 |
| Triethanolamine lactate | — | 6 | — | 6 |
| 1,3-butylene glycol | 3 | 3 | 3 | 3 |
| Preservative | 0.1 | 0.1 | 0.1 | 0.1 |
| Water | 78.9 | 72.9 | 76.4 | 70.4 |
| Maximum period (days) of stability at | | | | |
| 50°C | 3 | 7 | 45 | 90 |
| 42°C | 7 | 30 | 90 | 100 |
| 20°C | 15 | 45 | 100 | 120 |

From these results, it will be seen that the stability of the product containing neither lactate nor glutamate was very short-lived. The incorporation of lactate in amounts of from 6 to 20% by weight resulted in a slight improvement in stability, whereas the incorporation of glutamate produced a dramatic increase in stability, extending the period without apparent breakdown of the emulsion from a few days to at least 90 days, even at the highest storage temperature of 50°C.

Experiment 2

In this experiment, the stability of high internal phase emulsions containing inorganic electrolytes at different levels is compared with a similar product in which the electrolytes are replaced by a protein hydrolysate containing free amino acids, amino acid salts and hydrochlorides or by sodium glutamate.

As in Experiment 1, the stability of each product was assessed during storage at 50°C, 42°C, 20°C and also under conditions of freeze-thaw cycling (2 days at −20°C, then 2 days at +20°C, the cycling being repeated).

TABLE 2a

| | % by weight | | | | | | | | |
|---|---|---|---|---|---|---|---|---|---|
| | P1 | P2 | P3 | P4 | P5 | P6 | P7 | P8 | P9 |
| ISOPAR L®—ex Esso | 8 | 8 | 8 | 8 | 8 | 8 | 8 | 8 | 8 |
| HOSTAPHAT® KO 400 N—ex Hoechst | 3 | 3 | 3 | 3 | 3 | 3 | 3 | 3 | 3 |
| Para M*—ex Rhone Poulenc | 0.2 | 0.2 | 0.2 | 0.2 | 0.2 | 0.2 | 0.2 | 0.2 | 0.2 |
| Water | 88.8 | 87.8 | 89.8 | 87.8 | 89.8 | 87.8 | 89.8 | 87.8 | 89.8 |
| Ca $Cl_2$ | — | 1 | 4 | — | — | — | — | — | — |
| Mg $SO_4$ | — | — | — | 1 | 4 | — | — | — | — |
| Zn $SO_4$ | — | — | — | — | — | 1 | 4 | — | — |
| Na Cl | — | — | — | — | — | — | — | 1 | 4 |
| Maximum period (days) of stability at 50°C | 15 | 2 | 2 | 10 | 4 | 1 | 1 | 30 | 70 |
| 42°C | 15 | 2 | 2 | 10 | 10 | 1 | 1 | 85 | >100 |
| 20°C | 45 | 3 | 4 | 60 | 45 | 1 | 1 | 85 | >100 |
| Freeze-thaw (no. of cycles) | 1 | 1 | 1 | 1 | 1 | 0 | 0 | 2 | 2 |

*Methylparaben (=Methyl-p-hydroxy benzoate)

TABLE 2b

| | % by weight | | | | |
|---|---|---|---|---|---|
| | P10 | P11 | P12 | P13 | P14 |
| ISOPAR L®—ex Esso | 8 | 8 | 8 | 8 | 8 |
| HOSTAPHAT® KO 300 N—ex Hoechst | 3 | 3 | 3 | 3 | 3 |
| Para M*—ex Rhone Poulenc | 0.2 | 0.2 | 0.2 | 0.2 | 0.2 |
| Water | 82.8 | 87.8 | 84.8 | 87.8 | 84.8 |
| Na lactate pH 6 (50%) | 6 | — | — | — | — |
| Na glutamate | — | 1 | 4 | — | — |
| Collagen hydrolysate | — | — | — | 1 | 4 |
| Maximum period (days) of stability at 50°C | 16 | >100 | >100 | >100 | >100 |
| at 42°C | 27 | >100 | >100 | >100 | >100 |
| at 20°C | 45 | >100 | >100 | >100 | >100 |
| Freeze-thaw (no. of cycles) | 2 | 10 | >23 | 8 | 8 |

*Methylparaben (=Methyl-p-hydroxy benzoate)

From these results, it will be seen that the stability of the emulsions containing inorganic electrolytes is very short-lived, whereas that containing amino acids in the form of a protein hydrolysate or sodium glutamate is considerably extended, even at the higher storage temperature of 50°C.

Evidence for employing an amino acid or an amino acid salt at a concentration in the emulsion of from 0.5 to 25% by weight was provided by the following comparative experiment.

Experiment 3

In this experiment, emulsions were prepared, each containing sodium glutamate at different concentrations.

The emulsions had the following basic formulation:

| | Formulation (% w/w) |
|---|---|
| ISOPAR L® | 10 |
| HOSTAPHAT® KO 300N | 3 |
| PARA M* | 0.2 |
| Sodium glutamate | 0 to 30 |
| Water | to 100 |

*Methylparaben (=Methyl-p-hydroxy benzoate)

Sodium glutamate was employed at the following concentrations (% w/w): 0, 0.5, 1.5, 10, 25 and 30.

Samples of each emulsion were stored at 20°C, 42°C and 50°C, and were also subjected to freeze-thaw cycling as described in Experiment 2. They were examined daily for any signs of instability as shown by separation of oil or loss of viscosity. The results can be summarised as follows:

| Sodium glutamate % w/w | Storage time (days) without loss of stability at (°C) (maximum) | | | No. of freeze-thaw cycles without loss of stability |
|---|---|---|---|---|
| | 20 | 42 | 50 | |
| 0 | 15 | 10 | 10 | 1 |
| 0.5 | 60 | 50 | 45 | 1 |
| 1.5 | >100 | >100 | >100 | 2 |
| 10 | >100 | >100 | 85 | 1 |
| 25 | 45 | 30 | 18 | 1 |
| 30 | 30 | 25 | 15 | 1 |

Conclusion:

From these results, it is apparent that the stability of the emulsions containing the amino acid salt, sodium glutamate, at a concentration in the emulsion of from 0.5 to 25% by weight of the emulsion, was satisfactory for at least 18 days, even at the high storage temperature of 50°C. The results for the 1.5% and 10% by weight levels were superior. On the other hand, the stability of those samples containing no amino acid salt, or, at the upper end of the scale, more than 25% by weight of sodium glutamate, was very short lived.

Freeze-thaw stability was best at the 1.5% by weight level.

Evidence for employing an emulsifier at a concentration in the emulsion of from 0.5 to 10% by weight was provided by the following comparative experiment.

Experiment 4

In this experiment, emulsions were prepared, each containing an emulsifier at different concentrations.

The emulsions had the following basic formulation:

| | Formulation (% w/w) |
|---|---|
| ISOPAR L® | 10 |
| HOSTAPHAT® KO 300N* | 0.5 to 12 |
| PARA M[1)] | 0.2 |
| Sodium glutamate | 3 |
| Water | to 100 |

*The emulsifier was employed at the following concentrations (% w/w): 0.5, 2, 5, 10 and 12.

[1)]Methyl-p-hydroxy benzoate

Samples of each emulsion were stored at 20°C, 42°C and 50°C and were also subjected to freeze-thaw cycling as described in Experiment 2. They were examined daily for any signs of instability as shown by separation of oil or loss of viscosity. The results can be summarised as follows:

| Emulsifier % w/w | Storage time (days) without loss of stability at (°C) (maximum 3 months) | | | No. of freeze-thaw cycles without loss of stability |
|---|---|---|---|---|
| | 20 | 42 | 50 | |
| 0.5 | 55 | 40 | 10 | 1 |
| 2 | >100 | >100 | >100 | 2 |
| 5 | >100 | >100 | 90 | 2 |
| 10 | 40 | 30 | 20 | 1 |
| 12 | 40 | 25 | 10 | 1 |

Conclusion:

From these results, it is apparent that the stability of the emulsions containing an emulsifier at a concentration in the emulsion of from 0.5 to 10% by weight of the emulsion was satisfactory for at least 10 days, even at the high storage temperature of 50°C. The results for the 2% and 5% by weight levels were superior. On the other hand, the stability of those samples containing more than 10% by weight of the emulsion of emulsifier were very short-lived.

Freeze-thaw stability was best at the 2% and 5% by weight levels.

Evidence for employing an oil at a concentration of from 1 to 15% by weight was provided by the following comparative experiment.

Experiment 5

In this experiment, emulsions were prepared, each containing an oil at different concentrations.

The emulsions had the following basic formulation:

| | Formulation (% w/w) |
|---|---|
| Oil | 1 to 25 |
| Emulsifier | 1 to 3 |
| Para M | 0.2 |
| Sodium glutamate | 3 to 5 |
| Water | to 100 |

The individual formulations were as follows:

| | % w/w | | |
|---|---|---|---|
| | 5a | 5b | 5c |
| ISOPAR L | 1 | 2 | 3 |
| HOSTAPHAT KO 300 N | 3 | 3 | 3 |
| PARA M | 0.2 | 0.2 | 0.2 |
| Sodium glutamate | 3 | 3 | 3 |
| Water | 92.8 | 91.8 | 90.8 |

| | % w/w | | |
|---|---|---|---|
| | 5d | 5e | 5f |
| LYTOL | 15 | 24 | 25 |
| CREMOPHOR WO/A | 3 | 2 | 1 |
| PARA M | 0.2 | 0.2 | 0.2 |
| Sodium glutamate | 3 | 5 | 5 |
| Water | 78.8 | 68.8 | 68.8 |

Samples of each emulsion were stored at 20°C, 42°C and 50°C and were also subjected to freeze-thaw cycling as described in Experiment 2. They were examined daily for any signs of instability as shown by separation of oil or loss of viscosity. The results can be summarised as follows:.

| | Storage time (days) without loss of stability at (°C) (maximum 3 months) | | | No. of freeze-thaw cycles without loss of stability |
|---|---|---|---|---|
| Oil (% w/w) | 20 | 42 | 50 | |
| 1 | >100 | >100 | 45 | >23 |
| 2 | >100 | >100 | >100 | >23 |
| 3 | >100 | >100 | >100 | >23 |
| 15 | >100 | >100 | 90 | 10 |
| 24 | 40 | 30 | 18 | 2 |
| 25 | 15 | 10 | 5 | 1 |

Conclusion:

From these results, it is apparent that the stability of the emulsion containing an oil at a concentration in the emulsion of from 1 to 15% by weight of the emulsion was satisfactory for at least

Examples 1 & 2

Examples 1 and 2 illustrate moisturising lotions.

| | % w/w Ex. 1 | % w/w Ex. 2 |
|---|---|---|
| HOSTAPHAT® KO 300 N | 3 | — |
| IMWITOR® 780K | — | 3 |
| ISOPAR L® | 15 | 15 |
| Sodium glutamate | 2.5 | 2.5 |
| Triethanolamine lactate—50%: pH 5.5 (moisturising agent) | 6 | 6 |
| Para P(1) | 0.1 | 0.1 |
| 1,3-butylene glycol | 3 | 3 |
| Water | 70.4 | 70.4 |

(1)propyl p-hydroxybenzoate.

The oil was mixed with the emulsifier, the Para P added and dissolved at a temperature of 60—70°C. The aqueous phase containing the amino acid, the lactate and the glycol were heated gently at 45—50°C. Finally, the emulsion was prepared after cooling by emulsifying the aqueous phase with the oily phase to provide a water-in-oil emulsion at a temperature no higher than 50°C.

Example 3

An emulsion having the following formulation as a further example of a skin moisturising lotion for topical application, was prepared by the method described for Examples 1 and 2. It contained the following ingredients:

| | Ingredients | % w/w |
|---|---|---|
| O | HOSTAPHAT® KO 300 N—ex Hoechst | 3 |
| O | LYTOL®—ex Witco | 15 |
| O | Para P—ex Rhone Poulenc | 0.1 |
| A | Sodium pyrrolidone carboxylate (Na PC)1) (moisturising agent) | 4 |
| A | 1,3-butylene glycol | 3 |
| A | Collagen hydrolysate | 3 |
| A | Na glutamate | 4 |
| A | Par M2) | 0.2 |
| A | Water | 67.5 |
| A | Perfume | 0.2 |

1)Sodium pyrrolidone carboxylate (50% in water)
2)Methylparaben (=Methyl-p-hydroxy benzoate)
O Oily phase
A Aqueous phase

Examples 4 to 6

These emulsions illustrate the formulation of moisturising creams for topical application to the skin:

| | | Example 4 | Example 5 | Example 6 |
|---|---|---|---|---|
| O | HOSTAPHAT® KO 300 N—ex Hoechst | 3 | 3 | 3 |
| O | ISOPAR L®—ex Esso | 5 | — | — |
| O | PARLEAM®—ex Nichiyu | 5 | — | 5 |
| O | LYTOL®—ex Witco | — | 10 | — |
| O | WM1 oil—ex BP | — | — | 5 |
| O | PARA P1)—ex Rhone Poulenc | 0.1 | 0.1 | 0.1 |
| A | Na PC (50%)2) | 4 | 4 | 4 |
| A | 1,3-butylene glycol | 3 | 3 | 3 |
| A | Collagen hydrolysate | 3 | 3 | 3 |
| A | Na glutamate | — | 2 | 2 |
| A | PARA M—ex Rhone Poulenc | 0.2 | 0.2 | 0.2 |
| A | Water | 76.5 | 74.5 | 74.5 |
| A | Perfume | 0.2 | 0.2 | 0.2 |

1)Propyl-p-hydroxy benzoate
2)See Example 3
3)Methylparaben (=Methyl-p-hydroxy benzoate)

90 days, even at the high storage temperature of 50°C. The results for the 2% to 15% by weight levels were superior. On the other hand, the stability of those samples containing more than 24% by weight of the emulsion of oil were very shortlived.

Freeze-thaw stability was best at the 1 to 3% by weight levels.

Evidence for employing no more than 97% by weight of water in the emulsion was provided by the following comparative experiment.

Experiment 6

In this experiment, emulsions were prepared, each containing water at different concentrations. The emulsions had the following basic formulation:

| | Formulation (% w/w) |
|---|---|
| ISOPAR L® | 2 |
| HOSTAPHAT® KO 300 N | 1 to 2 |
| Sodium glutamate | 1 to 2 |
| Water | 94 to 98 |

The individual formulations were as follows:

| | % w/w | | | | |
|---|---|---|---|---|---|
| | 6a | 6b | 6c | 6d | 6e |
| ISOPAR L® | 2 | 2 | 2 | 1 | 1 |
| HOSTAPHAT® KO 300 N | 2 | 1 | 1 | 1 | 0.5 |
| Sodium glutamate | 2 | 2 | 1 | 1 | 0.5 |
| Water | 94 | 95 | 96 | 97 | 98 |

Samples of each emulsion were stored at 20°C, 42°C and 50°C and were also subjected to freeze-thaw cycling as described in Experiment 2. They were examined daily for any signs of instability as shown by separation of oil or loss of viscosity. The results can be summarised as follows:

| | Storage time (days) without loss of stability at (°C) (maximum 3 months) | | | No. of freeze-thaw cycles without loss of stability |
|---|---|---|---|---|
| Water % w/w | 20 | 42 | 50 | |
| 94 | >100 | >100 | >100 | >23 |
| 95 | >100 | >100 | 70 | 2 |
| 96 | >100 | 90 | 45 | 2 |
| 97 | >100 | 90 | 40 | 1 |
| 98 | 0 | 0 | 0 | 0 |

Conclusion:

From these results, it is apparent that the stability of the emulsion containing water at a concentration in the emulsion of no more than 95% by weight of the emulsion, was satisfactory for at least 70 days, even at the high storage temperature of 50°C. The result for the 94% by weight level was superior. On the other hand, the samples containing 98% by weight of the emulsion of water were completely unstable.

The invention is illustrated by the following examples:

Examples 7 to 9

These Examples also illustrate the formulation of moisturising creams for topical application to the skin:

| | | Example 7 | Example 8 | Example 9 |
|---|---|---|---|---|
| O | HOSTAPHAT® KO 300 N—ex Hoechst | 3 | 3 | 3 |
| | MARCHOL® 82—ex Esso | 5 | — | — |
| | ISOPAR® L—ex Esso | 5 | 5 | — |
| | PARLEAM®—ex Nichiyu | — | 5 | — |
| | LYTOL®—ex Witco | — | — | 10 |
| | CEREWAX L®—ex La Ceresine | 0.2 | 0.2 | 0.2 |
| | MODULAN®—ex Amerchol[1)] | 1 | 1 | 1 |
| | PARA P[2)]—ex Rhone Poulenc | 0.1 | 0.1 | 0.1 |
| A | Na PC (50%)[4)] | 4 | 4 | 4 |
| | 1,3-butylene glycol | 3 | 3 | 3 |
| | Collagen hydrolysate | 3 | 3 | 3 |
| | PARA M[3)]—ex Rhone Poulenc | 0.2 | 0.2 | 0.2 |
| | Water | 75.3 | 75.3 | 75.3 |
| | Perfume | 0.2 | 0.2 | 0.2 |

[1)] acetylated lanolin
[2)] Propyl-p-hydroxybenzoate
[3)] Methyl-p-hydroxybenzoate
[4)] see Example 3

Example 10

This example also illustrates the formulation of a moisturising cream for topical application to the skin:

| | Ingredients | % w/w |
|---|---|---|
| O | HOSTAPHAT® KO 300 N | 3 |
| | LYTOL® | 5 |
| | MARCHOL 82 | 5 |
| | CEREWAX L® | 0.2 |
| | MODULAN® | 1 |
| | PARA P[1)] | 1 |
| A | Na PC (50%)[2)] | 4 |
| | 1,3-butylene glycol | 3 |
| | Collagen hydrolysate | 3 |
| | Na glutamate | 2 |
| | PARA P | 0.2 |
| | Water | 73.3 |
| | Perfume | 0.2 |

Example 11

This example also illustrates the formulation of a moisturising cream for topical application to the skin:

| | Ingredients | % w/w |
|---|---|---|
| O | HOSTAPHAT KO 300 N | 3.5 |
| | ISOPAR L | 5 |
| | PARLEAM | 5 |
| | CEREWAX L® | 0.2 |
| | MODULAN® | 1 |
| | PARA P[1)] | 0.1 |
| A | Na PC (50%) | 4 |
| | Glycine | 5 |
| | PARA M[3)] | 0.2 |
| | Water | 72.8 |
| | Perfume | 0.2 |

[1)] Propyl-p-hydroxy benzoate
[2)] Methyl-p-hydroxy benzoate
[3)] see Example 3

Examples 12 & 13

These examples also illustrate the formulation of moisturising creams for topical application to the skin:

| | Ingredient | % w/w Example 12 | % w/w Example 13 |
|---|---|---|---|
| O | ARLACEL® 987—ex Atlas[1] | 2.5 | 2.5 |
| | Carnation mineral oil—ex Witco | 5 | 5 |
| | CETIOL® V—ex Henkel[2] | 5 | 5 |
| | PARA P[4] —Rhone Poulenc | 0.2 | 0.2 |
| | BENTONE® 38—ex National Lead[3] | 0.5 | 0.5 |
| A | Lactic—Na lactate mixture pH 5.5 | 5 | — |
| | Na PC (50%)[5] | — | 4 |
| | 1,3-butylene glycol | 3 | 3 |
| | Na glutamate | 1 | 1 |
| | PARA M[6] | 0.3 | 0.3 |
| | Water | 77.3 | 78.3 |
| | Perfume | 0.2 | 0.2 |

[1] Sorbitan isostearate
[2] Dodecyl oleate
[3] Organically modified montmorillonite clay
[4] Propyl-p-hydroxy benzoate
[5] see Example 3
[6] Methyl-p-hydroxy benzoate

Examples 14 & 15

These examples illustrate the formulation of light creams of topical application to the skin:

| | Ingredient | % w/w Example 14 | % w/w Example 15 |
|---|---|---|---|
| O | IMWITOR® 780K—ex Dynamit Nobel | 2.5 | — |
| | HOSTAPHAT® KO 300 N | — | 3 |
| | ISOPAR L® | 5 | — |
| | MARCHOL® 82 | 5 | 5 |
| | PARLEAM® | — | 5 |
| | CEREWAX® L | 0.3 | — |
| | MODULAN® | 1 | — |
| | PARA P[1] | 0.1 | 0.1 |
| A | 1,3-butylene glycol | — | 3 |
| | Na glutamate | 3 | — |
| | Collagen hydrolysate | — | 3 |
| | PARA M[2] | 0.2 | 0.2 |
| | Water | 82.7 | 80.5 |
| | Perfume | 0.2 | 0.2 |

[1] Propyl-p-hydroxy benzoate
[2] Methyl-p-hydroxy benzoate

Examples 16 & 17

These examples illustrate the formulation of sun protective creams for topical application to the skin.

| | Ingredient | % w/w Example 16 | % w/w Example 17 |
|---|---|---|---|
| O | ARLACEL® 987 (sorbitan isostearate) | 2.5 | — |
| | HOSTAPHAT® KO 300 N | — | 3 |
| | Carnation mineral oil | 5 | — |
| | CETIOL® V | 5 | — |
| | ISOPAR® L | — | 5 |
| | PARLEAM® | — | 5 |
| | BENTONE® 38 | 0.5 | — |
| | PARSOL MCX[1)] | 1 | 1 |
| | Para P[2)] | 0.2 | 0.1 |
| A | Na PC (50%)[3)] | 4 | 4 |
| | 1,3-butylene glycol | 3 | 3 |
| | Na glutamate | 0.5 | — |
| | Collagen hydrolysate | — | 3 |
| | Para M[4)] | 0.3 | 0.2 |
| | Water | 77.5 | 75.7 |

[1)]Ethyl hexyl-p-methoxy cinnamate
[2)]Propyl-p-hydroxy benzoate
[3)]see Example 3
[4)]Methyl-p-hydroxy benzoate

Example 18

This example illustrates the formulation of a cold cream for topical application to the skin:

| | Ingredients | % w/w |
|---|---|---|
| O | HOSTAPHAT® KO 300 N | 3 |
| | ISOPAR® L | 5 |
| | PARLEAM® | 5 |
| | PARA P[1)] | 0.1 |
| A | Na PC (50%)[2)] | 4 |
| | 1,3-butylene glycol | 3 |
| | Ethyl alcohol | 5 |
| | Collagen hydrolysate | 3 |
| | PARA M[3)] | 0.2 |
| | Water | 71.5 |
| | Perfume | 0.2 |

Example 19

This example illustrates the formulation of a non-greasy moisturising cream for topical application to the skin:

| | Ingredients | % w/w |
|---|---|---|
| O | HOSTAPHAT® KO 300 N | 3 |
| | ISOPAR L® | 5 |
| | PARLEAM® | 5 |
| | PARA P[1)] | 0.1 |
| A | Na PC (50%) | 4 |
| | Propylene glycol | 3 |
| | Collagen hydrolysate | 3 |
| | AEROSIL® 200[(1)] | 3 |
| | PARA M[3)] | 0.2 |
| | Water | 73.5 |
| | Perfume | 0.2 |

[(1)]Silica aerogel
[1)]Propyl-p-hydroxy benzoate
[2)]see Example 3
[3)]Methyl-p-hydroxy benzoate

**Claims**

1. A cosmetically and pharmaceutically acceptable water-in-oil emulsion comprising an amino acid or salt thereof or a mixture of amino acids and salts thereof in addition to water, an oil and an emulsifier, characterised in that:

(i) the amino acid component is water-soluble and forms from 0.5 to 25% by weight of the emulsion
(ii) the water forms from 60 to 95% by weight of the emulsion
(iii) the oil is a non-polar branched chain mineral oil and forms from 2 to 15% by weight of the emulsion and
(iv) the emulsifier is a liquid emulsifier having an HLB value of from 1 to 7 and forms from 0.5 to 10% by weight; of the emulsion

the emulsion having an aqueous phase comprising the amino acid component and water, the aqueous phase forming from 80 to 97% by volume of the emulsion, and an oily phase comprising the oil and emulsifier, the oily phase forming from 3 to 20% by volume of the emulsion.

2. An emulsion according to claim 1, in which the amino acid or salt thereof or a mixture of amino acids or salts thereof form from 1.5 to 10% by weight of the emulsion.

3. An emulsion according to claim 1, in which the emulsifier forms from 2 to 5% by weight of the emulsion.

4. A process for preparing a water-in-oil emulsion according to any preceding claim, which comprises the steps of

a) mixing a water-soluble amino acid component with water to provide an aqueous phase;
b) mixing the liquid emulsifier with the branched chain non-polar mineral oil to provide an oily phase; and
c) homogenising the aqueous phase and the oily phase to provide the water-in-oil emulsion.

**Patentansprüche**

1. Kosmetisch und pharmazeutisch annehmbare Wasser-in-Öl-Emulsion, umfassend eine Aminosäure oder deren Salz oder ein Gemisch von Aminosäuren und deren Salzen neben Wasser, einem Öl und einem Emulgator, dadurch gekennzeichnet, daß

(i) die Aminosäurekomponente wasserlöslich ist und 0,5 bis 25 Gew.-% der Emulsion bildet;
(ii) das Wasser 60 bis 95 Gew.-% der Emulsion bildet;
(iii) das Öl ein nicht-polares, verzweigtkettiges Mineral-öl ist und 2 bis 15 Gew.-% der Emulsion bildet; und
(iv) der Emulgator ein flüssiger Emulgator mit einem HLG-Wert von 1 bis 7 ist und 0,5 bis 10 Gew.-% der Emulsion bildet;

wobei die Emulsion eine wässrige Phase, die die Aminosäurekomponente und Wasser umfaßt, wobei die wässrige Phase von 80 bis 97 Vol.-% der Emulsion bildet, und eine ölige Phase, die das Öl und Emulgator umfaßt, wobie die ölige Phase 3 bis 20 Vol.-% der Emulsion ausmacht, aufweist.

2. Emulsion nach Anspruch 1, worin die Aminosäure oder deren Salz oder ein Gemisch von Aminosäuren oder deren Salzen von 1,5 bis 10 Gew.-% der Emulsion bilden.

3. Emulsion nach Anspruch 1, worin der Emulgator 2 bis Gew.-% der Emulsion bildet.

4. Verfahren zur Herstellung einer Wasser-in-Öl-Emulsion gemäß irgend einem vorhergehenden Anspruch, das die Schritte des a) Mischens einer wasserlöslichen Aminosäurekomponente mit Wasser zur Bildung einer wässrigen Phase; b) Mischens des flüssigen Emulgators mit dem verzweigtkettigen, nicht-polaren Mineralöl zur Bildung einer öligen Phase; und c) Homogenisierens der wässrigen Phase und der öligen Phase zur Bildung der Wasser-in-Öl-Emulsion umfaßt.

**Revendications**

1. Une émulsion eau-dans-huile acceptable cosmétiquement et pharmaceutiquement comprenant un amino acide ou un sel de celui-ci ou un mélange d'amino acides et de sels de ceux-ci en addition dans de l'eau, une huile et un émulsifiant, caractérisée en ce que:

(i) Le composant amino acide est soluble dans l'eau et forme de 0,5 à 25% en poids de l'émulsion;
(ii) L'eau forme de 60 à 95% en poids de l'émulsion;
(iii) l'huile est une huile minérale à chaîne ramifiée non polaire et forme de 2 à 15% en poids de l'émulsion; et

(iv) l'émulsifiant est une émulsifiant liquide possédant une valeur HLB comprise entre 1 et 7 et forme de 0,5 à 10% en poids de l'émulsion;

l'émulsion possedant une phase aqueous comprenant le composant amino acide et l'eau, la phase aqueuse formant de 80 à 97% en volume de l'émulsion, et une phase huileuse comprenant l'huile et l'émulsifiant, la phase huileuse formant de 3 à 20% en volume de l'émulsion.

2. Une émulsion selon la revendication 1, dans laquelle l'amino acide ou le sel de celui-ci ou un mélange d'amino acides et de sels de ceux-ci forment de 1,5 à 10% en poids de l'émulsion.

3. Une émulsion selon la revendication 1, dans laquelle l'émulsifiant forme de 2 à 5% en poids de l'émulsion.

4. Un procédé de préparation d'une émulsion eau-dans-huile selon l'une des revendications précédentes, qui comprend les étapes de a) mélange d'une composant amino acide soluble dans l'eau avec de l'eau pour fournir une phase aqueuse; b) mélange de l'émulsifiant liquide avec l'huile minérale non polaire à chaîne ramifiée pour fournir la phase huileuse; et c) homogénéisation de la phase aqueuse et de la phase huileuse pour fournir l'émulsion eau-dans-huile.